Europäisches Patentamt

European Patent Office

Office européen des brevets

(11) Publication number: **0 165 666**
**B1**

(12) **EUROPEAN PATENT SPECIFICATION**

(45) Date of publication of patent specification: **21.06.89**

(21) Application number: **85302474.3**

(22) Date of filing: **09.04.85**

(51) Int. Cl.⁴: **A 61 B 17/58**

(54) Intramedullary splint.

(30) Priority: **14.04.84 GB 8409758**

(43) Date of publication of application:
**27.12.85 Bulletin 85/52**

(45) Publication of the grant of the patent:
**21.06.89 Bulletin 89/25**

(84) Designated Contracting States:
**BE CH DE FR GB LI NL SE**

(56) References cited:
**DE-B-2 260 839**
**US-A-2 077 804**
**US-A-2 490 364**
**US-A-3 596 656**
**US-A-4 204 531**
**US-A-4 237 875**

(73) Proprietor: **Papagiannopoulos, George**
**Derby Royal Infirmary London Road**
**Derby DE1 2QY (GB)**

(72) Inventor: **Papagiannopoulos, George**
**Derby Royal Infirmary London Road**
**Derby DE1 2QY (GB)**

(74) Representative: **Wharton, Peter Robert et al**
**URQUHART-DYKES & LORD Beckett's Bank**
**Chambers 19 Cheapside**
**Bradford West Yorkshire BD1 4HR (GB)**

Courier Press, Leamington Spa, England.

## Description

This invention relates to an improved intramedullary compression "nail" or splint and in particular relates to such a splint for use with the so-called long bones.

The conventional external splinting of long bone fractures, of which fractures of the femur comprise a large proportion, is fraught with problems. These include morbility associated with long-term recumbency (particularly with older patients), and the inability to undergo full rehabilitation. The present surgical techniques only achieve rigid fixation of fermural shaft fractures in a very small proportion of cases. Patients are therefore unable to mobilise soon after surgery, and often have to undergo an extended period of hospitalisation until the fracture has healed. Early active mobilisation of patients aids healing of the fracture and of course avoids the disadvantages of long-term recumbency.

The invention seeks to provide means for the rigid fixation of the majority of long bone fractures, therefore allowing immediate active mobilization of the patient, with full weight-bearing, after surgery.

From US—A—2077804, there is known an apparatus for use with fractured bones which comprises an elongate member for connection to the bone, the elongate member having clamping means towards one end thereof which is movable between an operative and an inoperative position, means operable from the other end to control movement of the clamping means, and means operable from the said other end of compress the fracture surfaces when the elongate member is connected to the bone with the clamping means in the operative position, the clamping means comprising a pair of wings which, in the inoperative position, are recessed within the elongate member, and, in the operative position, project from opposite sides thereof to grip the bone.

The present invention provides an improved apparatus as disclosed in the characterizing part of claim 1.

Preferably, the elongate member is shaped to conform to the curvature of the bone to which it is to be connected.

Preferably also, the compressing means comprises a washer located at or near the said other end of the elongate member in a non-rotatable but axially movable manner. A member, such as nut, is threadably mounted at or near to said other end such that rotation thereof effects axial movement of the washer. The washer advantageously includes an insert of resilient material, and a plurality of spikes, or other gripping means, facing toward the said one of the elongate member.

The elongate mamber may comprise a tube within which is located an operating rod for the clamping means. The wings are pivotally attached to the bottom of the rod and are caused to project from the tube by the rod being forced downwardly by means of, for example, a screw at the said other end of the elongate member bearing on the rod.

The invention will be described further, by way of example, with reference to the accompanying drawings, in which:

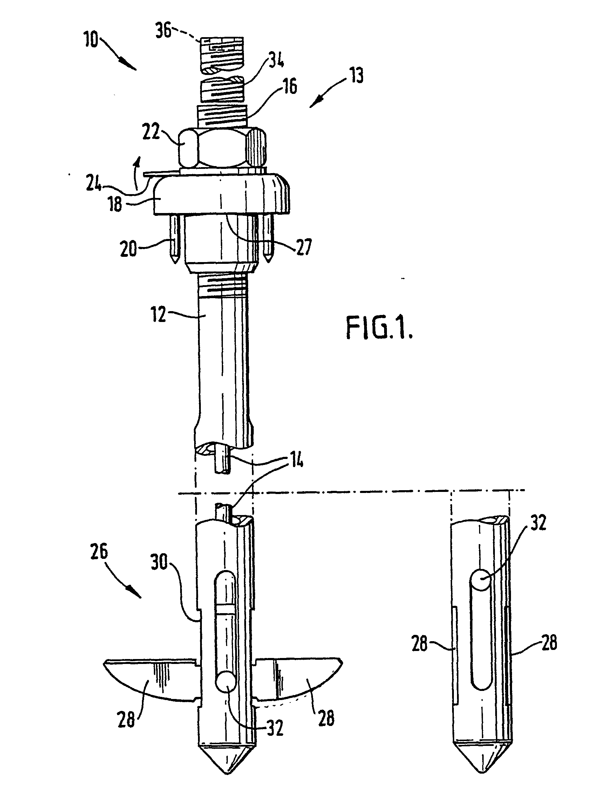

Figure 1 is side view, partly broken away, of an apparatus in accordance with the invention; and

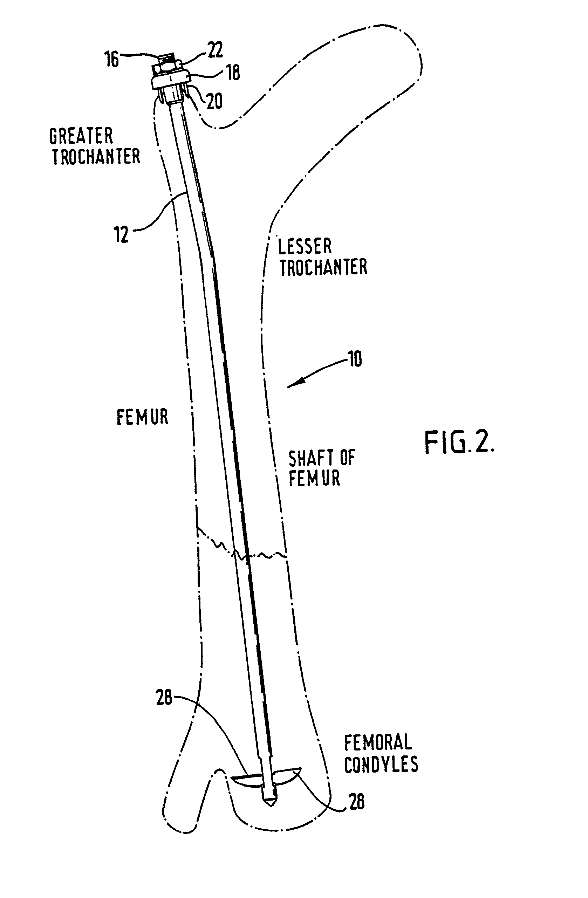

Figure 2 is diagrammatic view of the apparatus in place in a femur.

Referring to the drawings, an intramedullary splint generally designated 10 comprises an elongate member being a cylindrical closed tube 12, having one end 26 and an other end 13, within which is located a rod 14. The tube 12 is threaded at 16 and has two opposing flats (not shown). A washer 18 is mounted on the threaded portion 16 so as to be axially movable but held against rotation by the flats. The washer 18 is generally mushroom-shaped and carries a series of spikes 20 depending therefrom in the general direction the end 26 of the member 12.

A threaded nut 22 is positioned on the tube 12 above the washer 18, together with an associated lock-washer 24. Within the washer 18 is an insert 27 of a resilient material, e.g. silicone rubber.

At the end 26 of the tube 12 there are located clamping means comprising a pair of wings 28 mounted within slots 30 in the tube 12 and pivotally attached at 32 to the distal end of the rod 14. The upper end of the rod 14 is threaded to receive an extractor device (not shown) which can remove the rod from the tube if required. This upper threaded portion may also usefully receive and locate part of a special introducer device (also not shown) which both locks the rod (and therefore the wings) against inadvertent movement and gives the necessary leverage to insert the apparatus into a fractured bone. A rod operating screw 34 locates within the upper portion of the said other end of the tube which is threaded internally to receive the screw 34, as well as externally at 16. The screw 34 has a shaped, e.g. hexagonal, socket 36 to receive an Allen key in order to turn it so that it can bear against the upper portion of the rod 14 and drive it downwardly thereby causing the wings to project into their operative position.

In use, the wings are fully retracted and are held so by means of the introducer referred to above. The apparatus 10 is inserted, for a fractured femur, from the greater trochanter until its end 26 locates un the femural condyles, after the fracture has been reduced (i.e. the fractured surfaces brought together). The screw 34 is inserted and tightened thereby causing the wings 28 to project until they impinge upon cortical bone. This stabilises the lower end 26 of the splint 10 in the distal femur. The washer 18 is then fitted to the upper end of the tube 12 and the nut 22 tightened thereby causing the spikes 20 to engage the greater trochanter and clamp the upper and lower sections of the femur together, exerting pressure directly on the proximal fragments so that the fracture surfaces are compressed. The bone is thus stabilized against movement, and the patient can be mobilised rapidly. To prevent the nut 22 becoming loosened, e.g. by muscle movement, the end of the lock-washer 24 is bent up to lock the nut against rotation.

The tube 12 is shaped to conform to the normal curvature of the bone being supported. This may be ascertained by measurements, for instance on cadavers. Apart from the femur, the apparatus of the invention may be used with other long bones, both in humans and animals, such as the tibia or humerus. Naturally, the shape and length of the tube 12 may need to be adjusted, as may be configuration of the washer 18, but the principle of the device remains unchanged.

### Claims

1. Apparatus for use with fractured bones which comprises an elongate member (12) for connection to the bone, the elongate member having clamping means (28) at one end thereof which is movable between an operative and an inoperative position, means (34) operable from the other end to control movement of the clamping means, and means (22) operable from the said other end to compress the fracture surfaces when the elongate member (12) is connected to the bone with the clamping (28) means in the operative position, the clamping means comprising a pair of wings (28) which, in the inoperative position, are recessed within the elongate member (12) and, in the operative position, project from opposite sides thereof to grip the bone, characterised in that the wings (28) are pivotally attached (32) to the bottom of an operating rod (14) within the elongate member (12) whereby the wings are caused to move to the operative position, where they project from the elongate member (12), by the rod (14) being forced downwardly, and are held in that position by the rod (14) until required to be retracted to the inoperative position by the rod (14) being drawn upwardly by the means (34) operative to control the movement of the clamping means.

2. Apparatus as claimed in claim 1 in which the elongate member (12) is shaped to conform to the curvature of the bone to which it is to be connected.

3. Apparatus as claimed in either of claims 1 or 2 in which the compressing means comprises a washer (18) located at or near the said other end and in a non-rotatable but axially movable manner, co-operating with a threaded member (22) rotation of which moves the washer axially.

4. Apparatus as claimed in claim 3 in which the washer (18) has an insert of resilient material (29), and gripping means (20) facing the said one end of the elongate member (12).

5. Apparatus as claimed in any of claims 1 to 4 in which the elongate member (12) comprises a tube within which is located an operating rod (14) for the clamping means (28).

6. Apparatus as claimed in claim 1 in which the rod (14) is forced downwardly by means of a screw (34) within the said other end of the elongate member (12) bearing on the upper end of the (14) rod.

### Patentansprüche

1. Vorrichtung zur Verwendung bei Knochenbrüchen, mit einem länglichen Teil (12) zur Verbindung mit dem Knochen, wobei das länglichen Teil an seinem einen Ende eine Einspannvorrichtung (28) aufweist, die zwischen einer Arbeitsstellung und einer Ruhestellung hin- und herbewegbar ist, einer von dem anderen Ende des länglichen Teils aus bedienbaren Einrichtung (34) zur Steuerung der Bewegung der Einspannvorrichtung, und einer von diesem anderen Ende aus bedienbaren Einrichtung (22), mit denen die Bruchflächen zusammengepreßt werden, wenn das längliche Teil (12) mit dem Knochen verbunden und die Einspannvorrichtung (28) in der Arbeitsstellung ist, wobei die Einspannvorrichtung ein Paar Flügel (28) aufweist, die in der Ruhestellung in dem länglichen Teil (12) versenkt sind und in der Arbeitsstellung auf entgegengesetzten Seiten des Teils (12) herausragen, so daß sie den Knochen erfassen, dadurch gekennzeichnet, daß die Flügel (28) verschwenkbar am unteren Ende einer Schubstange (14) im Inneren das länglichen Teils (12) befestigt sind, so daß sie mit Hilfe der Stange (14) in die Arbeitstellung, in der sie aus dem länglichen Teil (12) herausragen, bewegt werden, wenn die Stange nach unten gedrückt wird, und durch die Stange (14) so lange in dieser Position gehalten werden, bis es erforderlich ist, sie durch die Stange (14), die mit Hilfe der Einrichtung (34) zur Steuerung der Bewegung der Einspannvorrichtung nach oben gezogen wird, in die Ruhestellung zurückzuziehen.

2. Vorrichtung nach Anspruch 1, in der das längliche Teil (12) so ausgebildet ist, daß es sich der Biegung des Knochens anpaßt, mit dem es verbunden werden soll.

3. Vorrichtung nach Anspruch 1 oder 2, in der die Preßvorrichtung ein Abdeckelement (18) aufweist, das an oder nahe dem anderen Ende nichtdrehbar, aber axial beweglich angeordnet ist und mit einem Gewindeteil (22) zusammenwirkt, durch dessen Drehung es axial bewegt wird.

4. Vorrichtung nach Anspruch 3, in der das Abdeckelement (18) einen Einsatz aus nachgiebigem Material (29) sowie Greifeinrichtungen (20) aufweist, die dem einen Ende des länglichen Teils (12) zugewändt sind.

5. Vorrichtung nach einem der Ansprüche 1 bis 4, in der das längliche Teil (12) eine Röhre umfaßt, in deren Inneren eine Schubstange (14) für die Einspannvorrichtung (28) angeordnet ist.

6. Vorrichtung nach Anspruch 1, in der die Stange (14) mittels einer Schraube (34) im Inneren des anderen Endes der Lagerung des länglichen Teils (12) an oberen Ende der Stange (14) nach unten gedrückt wird.

## Revendications

1. Dispositif utilisable avec des os fracturés, qui comprend un élément allongé (12) pour junction à l'os, l'élément allongé comportant des moyens de serrage (28), à une de ses extrémités, qui sont mobiles entre une position active et une position inactive, des moyens (34) manoeuvrables à partir de l'autre extrémité pour commander le mouvement des moyens de serrage, et des moyens (22) manoeuvrables à partir de ladite autre extrémité pour comprimer les surfaces de la fracture lorsque l'élément allongé (12) est relié à l'os et que les moyens de serrage (28) sont dans la position active, les moyens de serrage comprenant deux ailes (28) qui, dans la position inactive, sont rétractées à l'intérieur de l'élément allongé (12) et, dans la position active, font saillie de part et d'autre de cet élément pour saisir l'os, caractérisé en ce que les ailes (28) sont attachées de façon pivotante (32) à la partie inférieure d'une tige de manoeuvre (14) placée dans l'élément allongé (12) de sorte que les ailes sont amenées à la position active, dans laquelle elles font saillie par rapport à l'élément allongé (12), par la tige (14) poussée vers le bas et elles sont maintenues dans cette position par la tige (14) jusqu'à ce qu'il soit nécessaire de les rétracter à la position inactive par traction de la tige (14) vers le haut à l'aide des moyens (34) qui commandent le mouvement des moyens de serrage.

2. Dispositif suivant la revendication 1, dans léquel l'élément allongé (12) à une configuration conforme à la courbure de l'os auquel il soit être fixé.

3. Dispositif suivant la revendication 1 ou 2, dans lequel les moyens de compression comprennent une rondelle (18) placée à ladite autre extrémité ou près de celle-ci, d'une manière non tournante mais axialement mobile, cette rondelle coopérant avec un organe fileté (22) dont la rotation déplace la rondelle axialement.

4. Dispositif suivant la revendication 3, dans lequel la rondelle (18) comprend un insert en matière élastique (29) et des moyens de prime (20) tournés vers ladite première extrémité de l'élément allongé (12).

5. Dispositif suivant l'une quelconque des revendications 1 à 4, dans lequel l'élément allongé (12) comprend un tube à l'intérieur duquel est logée une tige de manoeuvre (14) pour les moyens de serrage (28).

6. Dispositif suivant la revendication 1, dans lequel la tige (14) est poussée vers le bas au moyen d'une vis (34) logée dans ladite autre extrémité de l'élément allongé (12) et appuyant sur l'extrémité supérieure de la tige (14).

FIG.1.

FIG.2.